# EUROPEAN PATENT SPECIFICATION

(11) **EP 2 569 007 B1**
(45) Date of publication and mention of the grant of the patent: **14.10.2015**
(21) Application number: 11720079.0
(22) Date of filing: 10.05.2011
(51) Int. Cl.: A61K 39/02

(54) **VACCINE AGAINST MYCOPLASMA HYOPNEUMONIAE, SUITABLE FOR ADMINISTRATION IN THE PRESENCE OF MATERNALLY DERIVED ANTIBODIES**
IMPFSTOFF GEGEN MYCOPLASMA HYOPNEUMONIAE, GEEIGNET ZUR VERABREICHUNG IM BEISEIN VON AUS DER MUTTER ÜBERTRAGENEN ANTIKÖRPERN
VACCIN CONTRE L'HYOPNEUMONIE À MYCOPLASMA ADAPTÉ À UNE ADMINISTRATION EN PRÉSENCE D'ANTICORPS MATERNELS

(30) Priority: 11.05.2010 US 333502 P; 11.05.2010 EP 10162580
(43) Date of publication of application: 20.03.2013
(73) Proprietor: Intervet International B.V., 5831 AN Boxmeer (NL)
(72) Inventor: WITVLIET, Maarten Hendrik, 5831 AN Boxmeer (NL)
(74) Representative: Intervet International B.V.
(86) International application number: PCT/EP2011/057463
(87) International publication number: WO 2011/141443

(56) References cited:
- WO-A2-2007/103042
- US-A1- 2006 233 831
- JONES GARY F ET AL: "Intradermal vaccination for Mycoplasma hyopneumoniae", SWINE HEALTH AND PRODUCTION, DES MOINES, IA, US, vol. 13, 1 January 2005 (2005-01-01), pages 19-27, XP009138320, ISSN: 1066-4963
- CHRISTOPER C L CHASE ET AL: "Needle-free injection technology in swine: Progress toward vaccine efficacy and pork quality", SWINE HEALTH AND PRODUCTION, DES MOINES, IA, US, vol. 16, 1 September 2008 (2008-09-01), pages 254-261, XP009138358, ISSN: 1066-4963
- HOUSER TERRY A ET AL: "Effectiveness of transdermal, needle-free injections for reducing pork carcass defects", MEAT SCIENCE, ELSEVIER SCIENCE, GB LNKD- DOI:10.1016/J.MEATSCI.2004.03.016, vol. 68, no. 2, 1 October 2004 (2004-10-01), pages 329-332, XP002544853, ISSN: 0309-1740
- MERIAL: "SwiVaxTM-MH Derma-Vac N. Targeted Immune Response", INTERNET CITATION, 7 September 2009 (2009-09-07), pages 1-2, XP002544854, Retrieved from the Internet: URL:http://www.newportlabs.com/LinkClick.a spx?fileticket=aWlqR%2Ffe6MM%3D &tabid=488 [retrieved on 2009-09-07]
- MAES ET AL: "Control of Mycoplasma hyopneumoniae infections in pigs", VETERINARY MICROBIOLOGY, ELSEVIER BV, NL LNKD- DOI:10.1016/J.VETMIC.2007.09.008, vol. 126, no. 4, 4 December 2007 (2007-12-04), pages 297-309, XP022374563, ISSN: 0378-1135
- DREXLER C S ET AL: "Efficacy of combined porcine reproductive and respiratory syndrome virus and Mycoplasma hyopneumoniae vaccination in piglets", VETERINARY RECORD, BRITISH VETERINARY ASSOCIATION, LONDON, GB, vol. 166, no. 3, 16 January 2010 (2010-01-16), pages 70-74, XP009138359, ISSN: 0042-4900
- HODGINS DOUGLAS C ET AL: "Influence of age and maternal antibodies on antibody responses of neonatal piglets vaccinated against mycoplasma hyopneumoniae", SWINE HEALTH AND PRODUCTION, DES MOINES, IA, US, vol. 12, 1 October 2004 (2004-10-01), pages 10-16, XP009138331, ISSN: 1066-4963
- MARTELLI P ET AL: "Antibody response to Mycoplasma hyopneumoniae infection in vaccinated pigs with or without maternal antibodies induced by sow vaccination", JOURNAL OF VETERINARY MEDICINE. SERIES B - ZENTRALBLATT FUER VETERINAERMEDIZIN. REINE B, PAUL PAREY, BERLIN, DE LNKD- DOI:10.1111/J.1439-0450.2006.00952.X, vol. 53, no. 5, 1 June 2006 (2006-06-01), pages 229-233, XP009138302, ISSN: 0931-1793 [retrieved on 2006-05-24]

## Description

The present invention concerns a vaccine comprising inactivated *Mycoplasma hyopneumoniae* antigens for use in a method to actively protect an animal against a disorder arising from an infection with *Mycoplasma hyopneumoniae,* even when administered in the presence of maternally derived antibodies directed against *Mycoplasma hyopneumoniae.*

Mycoplasmal pneumonia of swine caused by the bacterial pathogen *Mycoplasma hyopneumoniae* (also abbreviated Mhyo) is a widespread chronic respiratory disease in pigs. Especially young piglets are vulnerable to this, non-fatal, disease. Enzootic pneumonia is a chronic disease that results in poor feed conversion and stunted growth. The disease is highly contagious and transmission is usually through direct contact with infected respiratory tract secretions, e.g. in the form of infected droplets after coughing/sneezing. An important problematic consequence of this disease is that it predisposes for all kinds of secondary infections of the respiratory system, which may cause a low average daily growth or even death. It is estimated that e.g. in the USA, 99% of all pig farms are infected with *Mycoplasma hyopneumoniae.* Yearly losses are estimated to be between 100 and 300 million dollars.

Numerous vaccines to protect an animal against an infection with *Mycoplasma hyopneumoniae* are known in the art. Effective vaccines are described, comprising a whole or partial cell inactivated or modified live preparation, a subunit vaccine or a nucleic acid or DNA vaccine. Commercially available vaccines such as Porcilis® M HYO (available from Intervet Schering-Plough Animal Health, Boxmeer, The Netherlands) are in many cases based on whole cell inactivated preparations (bacterins).

It is known that young piglets, even in the presence of maternally derived antibodies (MDA) against *Mycoplasma hyopneumoniae* may be (partly) protected when vaccinated intramuscularly with an Mhyo vaccine (Drexler et al in Veterinary Record, British Veterinary Association, London, Vol. 166, No. 3, 16 January 2010, pp 70-74. However, protection might be improved, in particular after a single vaccination in the presence of MDA's.

To this end it has been found that intradermal application of an Mhyo vaccine may induce improved active protection when compared to intramuscular vaccination. This allows at least an improved active protection against Mhyo, which can be induced in very young animals, without leaving a window wherein the animals are hardly protected against Mhyo, and without running a substantial risk that there is no take of the vaccine. The invention also pertains to the use of *Mycoplasma hyopneumoniae* antigens for the manufacture of a vaccine for intradermal application to an animal that has maternally derived antibodies against *Mycoplasma hyopneumoniae* in its serum, and to a method of actively protecting an animal that has maternally derived antibodies against *Mycoplasma hyopneumoniae,* against a disorder arising from an infection with *Mycoplasma hyopneumoniae.*

It is noted that Jones has described (Journal of Swine Health and Production, Vol. 13, No. 1, pp 19-27) that intradermal application of an Mhyo vaccine in 13 weeks old pigs leads to higher antibody titers than intramuscular application of the same vaccine. However, Jones also recognized that antibody titers do not correlate with actual protection. A comparison between actual protection obtained after intradermal or intramuscular application was not made. Indeed, Jones concludes only that intradermal application of an Mhyo vaccine "is feasible and protective".

It is also noted that WO 2007/103042 describes the intradermal application of an Mhyo vaccine. However, to test the efficacy of the vaccine, a low *Mycoplasma hyopneumoniae* incidence herd was used (see page 24, lines 19-20). No results in MDA positive animals are described, let alone a comparison between protection obtained after intramuscular and intradermal application.

A vaccine in the sense of this invention is a constitution suitable for application to an animal, comprising one or more antigens such as attenuated or killed micro-organisms and/or subunits thereof, or any other substance such as a metabolite of the microorganism, in an immunologically effective amount (i.e. capable of stimulating the immune system of the target animal sufficiently to at least reduce the negative effects of a challenge of the wild-type micro-organisms), typically combined with a pharmaceutically acceptable carrier such as a liquid containing water, which upon administration to the animal induces an immune response for treating a disease or disorder, i.e. aiding in preventing, ameliorating or curing the disease or disorder (for example coughing, dyspnoea and diarrhoe) due to the infection. In general, a vaccine can be manufactured by using art-known methods that basically comprise admixing the antigens (or a composition containing the antigens) with the pharmaceutically acceptable carrier. The antigens stimulate the immune system of the target animal sufficiently to at least reduce the negative effects of a post-vaccination challenge (for example by natural infection) with wild-type micro-organisms. Optionally other substances such as adjuvants, stabilisers, viscosity modifiers or other components are added depending on the intended use or required properties of the vaccine.

In an embodiment, the vaccine induces active protection after a single vaccination. Surprisingly it was found that even in the presence of maternally derived antibodies directed against Mhyo, it is possible to induce active protection against an infection with Mhyo, when the vaccine is administered intradermally. This means that even for prolonged protection, for example throughout fattening, a booster vaccination can be dispensed with.

In an embodiment the vaccine is for administration to animals between 10 and 18 days of age. It was found that even in MDA positive animals as young as 10 days, which animals have high titres of maternally derived antibodies, it is possible to induce an adequate active protection against an infection with Mhyo. If not administered substantially after 18 days of age, this means that the combined protection of maternally derived antibodies (passive protection during approximately the first 3-4 weeks of age) and an actively induced immune response (active protection during 1-6 months of age) may provide a virtually complete protection against an infection with Mhyo.

The antigens in the vaccine comprise inactivated *Mycoplasma hyopneumoniae* antigens Inactivated antigens, i.e. antigens other than live *Mycoplasma hyopneumoniae* bacteria (such as bacterins, subunits, etc.) are safe and have proven to provide an adequate immune response in pigs. In particular when adjuvant is used that comprises vitamin E acetate (also known as α-tocopheryl acetate), very good protection can be obtained in young animals having MDA's, even after a single intradermal vaccination.

The invention will now be further explained using the following examples.

### General trial set-up

A trial was carried out following a randomised and partially blinded design. On a farrow-to-finish farm, having no history of vaccination against Mhyo, and with a consistent presence of Mhyo infection, 1026 piglets (about 100 litters, piglets showing signs of disease were excluded) were randomly divided into three groups. One group (called "IM") was vaccinated intramuscularly with a vaccine comprising the same Mhyo antigens as the commercially available vaccine Porcilis M Hyo. The antigens were formulated in a twin emulsion adjuvant called "X". This adjuvant is a mixture of 5 volume parts of adjuvant "A" and 1 volume part of adjuvant "B". Adjuvant "A" consists of mineral oil droplets (Marcol 52) with an approximate average (volume weighed) size around 1 µm, stabilised with Tween 80 in water. Adjuvant "A" comprises 25 weight % of the mineral oil and 1 weight % of the Tween. Rest is water. Adjuvant "B" consists of droplets of biodegradable vitame E acetate with an approximate average (volume weighed) size of 400 nm, stabilised also with Tween 80. The adjuvant "B" comprises 15 weight% of vitamine E acetate and 6 weight % of Tween 80, rest is water. A second group (called "ID") was vaccinated intradermally with the same vaccine (but more concentrated), and a third group (called "Control") received solely the adjuvant by intramuscular injection. A single vaccination was given at an age of 10-18 days.

At admission, the piglets were tested for the presence of maternally derived antibodies against Mhyo. The piglets were individually weighed at admission and shortly before slaughter (at an age of about 150 days). In the slaughterhouse the lungs were scored for lung lesions typical for Mhyo infection and pleuritis. Morbidity (disease being defined as the perceived need for individual treatment) and mortality were recorded throughout the trial. Primary parameters for efficacy of the vaccines were the mean lung lesion score and the average daily weight gain (ADG). The morbidity, mortality and pleuritis lesions were considered secondary parameters of efficacy.

### Specific methods

### Vaccination and challenge

The reference vaccine was Porcilis® M HYO, a common type of Mhyo vaccine, comprising inactivated *Mycoplasma hyopneumonia* bacteria. Since this commercially available vaccine is a 2-shot vaccine and the present set-up is a single shot vaccination, the current vaccine was concentrated with respect to Porcilis® M HYO to have a higher antigenic content per ml vaccine. The IM group received 2 ml of a 5 times concentrated vaccine behind the left ear. The ID group received 0.2 ml of a 30 times concentrated vaccine in the upper part of the body, mainly in the neck behind the ears using a needleless intradermal injector as described in EP 1 515 763. The net effect was that the animals in the ID group received about 60% of the antigenic mass when compared to the animals in the IM group. Moreover, one has to realise that with a so-called intradermal administraton device such as the device known from EP 1 515 763, only a part of this antigenic mass is actually deposited in the dermis, the main part being deposited in underlying muscle tissue. The control group received 2 ml of the adjuvant only, injected intramuscularly behind the left ear.

The infection source was the natural challenge present on the farm, and infection was by natural exposure.

### Measurement of the effects

The presence and severity of the lung lesions associated with enzootic pneumonia were assessed according to the commonly known method by Goodwin and Whittlestone. For each of the seven pulmonary lobes, the proportion of the surface with signs of Mhyo associated inflammation was estimated. These proportions were multiplied with the weighing factors listed below in Table 1 and added up to obtain the total lesion score.

**Table 1**

| | Weighing factor |
|---|---|
| Apical lobes | 10 |
| Diaphragmatic lobes | 5 |
| Cardiac lobe | 10 |
| Accessory lobe | 5 |

The average daily weight gain (ADG) was calculated by dividing the weight gain from vaccination to slaughter, divided by the duration of that period (on average about 136 days).

The person who scored the lungs for pneumonic lesions also scored pleuritis lesions. The presence was scored as follows. One (1), when no lesions were present; Two (2), when topical lesions (spots) were present and Three (3) when larger adhesions were present.

For serology, at admission and slaughter blood samples were taken from 30 pigs per experimental group. These samples were tested for antibodies by ELISA (CHECKIT-Hyoptest II, IDEXX).

### Results

At admission, 92% of the piglets were seropositive for Mhyo antigens, which shows that almost all animals had maternally derived antibodies. There average age was 13.4 days, the youngest animals being 10 days of age, the oldest one 18 days. In a preceding trial with MDA negative animals (SPF pigs, experimental challenge with wild-type Mhyo bacteria) intradermal vaccination at an age of about 2 weeks gave no better efficacy results than intramuscular vaccination (data not shown). In the MDA positive pigs of the present trial however, a significant improved efficacy was found in the group that received the vaccine intradermally. The mean lung lesion scores (about 315 lungs per group) at the time of slaughter are presented in Table 2. The effect on the treatment of the lesion score was statistically significant (P<0.0001) according to Wicoxons Rank Sum test.

**Table 2**

| | IM | ID | Control |
|---|---|---|---|
| Mean lesion score | 18.4 | 14.1 | 19.5 |

The growth of the pigs is depicted in Table 3, showing the ADG (in grams) between the moment of vaccination and slaughter at an age of approximately 150 days. The results of the ID and IM group were compared using Tukey's method for multiple comparisons. It appeared that the weight gain of the ID group was significantly higher (P<0.05).

**Table 3**

| | IM | ID | Control |
|---|---|---|---|
| ADG | 622 | 640 | 617 |

The result for pleuritis lesions are depicted in Table 4. In the table, the number of pigs per score type are given.

**Table 4**

| | IM | ID | Control |
|---|---|---|---|
| 1 (no lesions) | 292 | 308 | 274 |
| 2 (spots) | 19 | 12 | 28 |
| 3 (larger adhesions) | 4 | 2 | 6 |

The incidence of clinical disease (morbidity) during the nursery period was about 28% in all three groups. During the finishing period however, the morbidity was 21 % in the control group, 17% in the IM group and merely 8% in the ID group (statistically significant, P=0.0007, according to the 2x2 contigency tables by chi square test). The signs "cough" and/or "dyspnoea" were recorded in 76% of the infected animals. Mortality was slightly different for the three groups: 4.6% for the controls, 2.2% for the IM group and 1.2% for the ID group. The fact that in seven parameters that are indicative for actual protection ("mean lesion score", "ADG", "lesions", "spots", "larger adhesions", "morbidity" and "mortality"), intradermal vaccination scores better than intramuscular vaccination means that there is a certainty of more than 99% (P = 1-(½)⁷= 0.993) that intradermal vaccination provides better protection in MDA positive piglets.

### Conclusion

Intradermal vaccination of animals having maternally derived antibodies against Mhyo, in particular with an inactivated vaccine (such as for example Porcilis Mhyo™; but any other inactivated vaccine, comprising e.g. killed bacteria and/or parts thereof either purified from Mhyo bacteria or recombinantly expressed, will provide comparable results given the fact that inactivated vaccines in principle elicit the same type of immune response), provides significantly better protection than intramuscular vaccination. In particular, the average daily weight gain, and thus the obtainable live body weight at the end of the finishing period benefits from this new administration regime for seropositive animals.

## Claims

1. A vaccine comprising inactivated *Mycoplasma hyopneumoniae* antigens for intradermal application to an animal that has maternally derived antibodies directed against *Mycoplasma hyopneumoniae,* for use in a method to actively protect the animal against a disorder arising from an infection with *Mycoplasma hyopneumoniae.*

2. A vaccine for use according to claim 1, **characterised in that** it induces active protection after a single vaccination.

3. A vaccine for use according to any of the preceding claims, **characterised in that** it is for administration to animals between 10 and 18 days of age.

4. A vaccine for use according to claim 1, **characterised in that** the vaccine comprises vitamin E acetate as an adjuvant.

5. Use of inactivated *Mycoplasma hyopneumoniae* antigens for the manufacture of a vaccine for intradermal application to an animal that has maternally derived antibodies against *Mycoplasma hyopneumoniae* in its serum, to protect the animal against a disorder arising from an infection with *Mycoplasma hyopneumoniae.*

## Patentansprüche

1. Impfstoff, umfassend inaktivierte *Mycoplasmahyopneumoniae-Antigene,* zur intradermalen Anwendung bei einem Tier, das gegen *Mycoplasma hyopneumoniae* gerichtete Antikörper mütterlicher Abstammung aufweist, zur Verwendung bei einem Verfahren zum aktiven Schutz des Tiers gegen eine sich aus einer Infektion mit *Mycoplasma hyopneumoniae* ergebende Störung.

2. Impfstoff zur Verwendung nach Anspruch 1, **dadurch gekennzeichnet, dass** er nach einer einzigen Impfung aktiven Schutz induziert.

3. Impfstoff zur Verwendung nach einem der vorhergehenden Ansprüche, **dadurch gekennzeichnet, dass** er zur Verabreichung an Tiere in einem Alter zwischen 10 und 18 Tagen vorgesehen ist.

4. Impfstoff zur Verwendung nach Anspruch 1, **dadurch gekennzeichnet, dass** der Impfstoff als Adjuvans Vitamin-E-acetat umfasst.

5. Verwendung von inaktivierten *Mycoplasma-hyopneumoniae-Antigenen* zur Herstellung eines Impfstoffs zur intradermalen Anwendung bei einem Tier, das in seinem Serum gegen *Mycoplasma hyopneumoniae* gerichtete Antikörper mütterlicher Abstammung aufweist, zum Schutz des Tiers gegen eine sich aus einer Infektion mit *Mycoplasma hyopneumoniae* ergebende Störung.

## Revendications

1. Vaccin comprenant des antigènes de *Mycoplasma hyopneumoniae* inactivés pour application intradermique à un animal qui des anticorps d'origine maternelle dirigés contre *Mycoplasma hyopneumoniae,* pour utilisation dans un procédé pour protéger activement l'animal contre un trouble dû à une infection par *Mycoplasma hyopneumoniae.*

2. Vaccin pour utilisation selon la revendication 1, celui-**caractérisé en ce qu'**il induit une protection active après une vaccination unique.

3. Vaccin pour utilisation selon l'une quelconque des revendications précédentes, **caractérisé en ce qu'**il est pour administration à des animaux à un âge compris entre 10 et 18 jours.

4. Vaccin pour utilisation selon la revendication 1, **caractérisé en ce que** le vaccin comprend de l'acétate de vitamine E en tant qu'adjuvant.

5. Utilisation d'antigènes de *Mycoplasma hyopneumoniae* inactivés pour la fabrication d'un vaccin pour application intradermique à un animal qui des anticorps d'origine maternelle dirigés contre *Mycoplasma hyopneumoniae* dans son sérum, pour protéger l'animal contre un trouble dû à une infection par *Mycoplasma hyopneumoniae.*
